(19) Europäisches Patentamt
European Patent Office
Office européen des brevets

(11) Veröffentlichungsnummer: **0 037 497**
**B1**

(12) # EUROPÄISCHE PATENTSCHRIFT

(45) Veröffentlichungstag der Patentschrift: **23.11.83**

(51) Int. Cl.³: **C 07 F 9/65,** A 01 N 57/16

(21) Anmeldenummer: **81102104.7**

(22) Anmeldetag: **20.03.81**

(54) **Pyrazol-5-yl-(thio)(thiol)phosphor(phosphon)säureester, Verfahren zu ihrer Herstellung und ihre Verwendung zur Bekämpfung von Schädlingen.**

(30) Priorität: **05.04.80 DE 3013291**

(43) Veröffentlichungstag der Anmeldung: **14.10.81 Patentblatt 81/41**

(45) Bekanntmachung des Hinweises auf die Patenterteilung: **23.11.83 Patentblatt 83/47**

(84) Benannte Vertragsstaaten: **BE DE FR GB NL**

(56) Entgegenhaltungen:
**AT - B - 184 580**
**DE - A - 2 603 215**
**DE - A - 2 639 258**
**US - A - 3 010 969**

**Die Akte enthält technische Angaben, die nach dem Eingang der Anmeldung eingereicht wurden und die nicht in dieser Patentschrift enthalten sind.**

(73) Patentinhaber: **BASF Aktiengesellschaft, Carl-Bosch-Strasse 38, D-6700 Ludwigshafen (DE)**

(72) Erfinder: **Loeffler, Hans-Peter, Dr., Defreggerstrasse 14, D-6700 Ludwigshafen (DE)**
Erfinder: **Hutmacher, Hans-Martin, Dr., Neckarpromenade 16, D-6800 Mannheim (DE)**
Erfinder: **Adolphi, Heinrich, Dr., Kalmitweg 11, D-6703 Limburgerhof (DE)**

**EP 0 037 497 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Pyrazol-5-yl(thio)(thiol)phosphor(phosphon)säureester, Verfahren zu ihrer Herstellung und ihre Verwendung zur Bekämpfung von Schädlingen

Die vorliegende Erfindung betrifft Pyrazol-5-yl(thio)(thiol)phosphor(phosphon)säureester, Verfahren zu ihrer Herstellung, Schädlingsbekämpfungsmittel, die diese Phosphorsäurederivate als Wirkstoffe enthalten, sowie Verfahren zur Bekämpfung von Schädlingen mit diesen Wirkstoffen.

Es ist bereits bekannt, dass 1-Phenyl-3-alkylpyrazol-5-yl(thio)phosphorsäureester sich zur Bekämpfung von Schädlingen, insbesondere von Aphiden und Akariden, eignen (AT-PS Nr. 184580). Ausserdem wurden 1-Phenyl-4-alkoxy(alkylmercapto)pyrazol-5-yl(di)thiophosphorsäureester als insektizid und akarizid wirksam beschrieben (DE-A Nr. 2603215).

Es wurde gefunden, dass Pyrazol-5-yl(thio)-(thiol)phosphor(phosphon)säureester der Formel I:

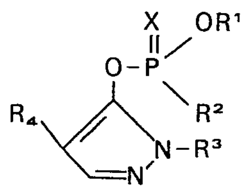

(I)

in der

X für Sauerstoff oder Schwefel,

$R^1$ für Alkyl mit 1 bis 4 Kohlenstoffatomen,

$R^2$ für Methyl, Äthyl, Phenyl oder für gegebenenfalls durch Halogen oder Alkoxy mit 1 bis 3 Kohlenstoffatomen substituiertes Alkoxy oder Alkylthio mit 1 bis 5 Kohlenstoffatomen,

$R^3$ für Wasserstoff, Alkyl mit 1 bis 6 Kohlenstoffatomen, Cycloalkyl mit 3 bis 6 Kohlenstoffatomen oder Phenyl, das durch Chlor, Brom, Methyl oder Alkoxy mit 1 bis 3 Kohlenstoffatomen substituiert sein kann, und

$R^4$ für Alkyl mit 1 bis 4 Kohlenstoffatomen stehen,

sich zur Bekämpfung von Schädlingen, insbesondere von Insekten und Spinnentieren, eignen. Sie sind in ihrer Wirkung bekannten 1-Phenyl-3-alkylpyrazol-5-yl(thio)phosphorsäureestern überlegen.

$R^1$ in Formel I steht für einen unverzweigten oder verzweigten Alkylrest mit 1 bis 4 Kohlenstoffatomen, wie Methyl, Äthyl, Propyl- und Butylreste, vorzugsweise für Methyl und Äthyl; $R^2$ bedeutet Methyl, Äthyl, Phenyl oder einen Alkoxy- oder Alkylthiorest mit 1 bis 5 Kohlenstoffatomen, der durch Halogen Alkoxy mit 1 bis 3 Kohlenstoffatomen substituiert sein kann, beispielsweise Methoxy, Äthoxy, Propoxy, Butoxy, Methylthio, Äthylthio, n-Propylthio, sec.-Butylthio, Isopropylthio, Isobutylthio, 2-Ätoxyäthylthio, 2-Methoxyäthylthio, 3-Chlor-n-propylthio.

$R^3$ bedeutet ausser Wasserstoff unverzweigtes oder verzweigtes Alkyl mit 1 bis 6 Kohlenstoffatomen, vorzugsweise mit 1 bis 3 Kohlenstoffatomen, beispielsweise Methyl, Äthyl, Isopropyl, Cycloalkyl mit 3 bis 6 Kohlenstoffatomen, vorzugsweise Cyclohexyl oder Phenyl, das durch Chlor, Brom, Methyl oder Alkoxy mit 1 bis 3 Kohlenstoffatomen, vorzugsweise Methoxy oder Äthoxy, einfach oder mehrfach substituiert sein kann, $R^4$ bedeutet unverzweigtes oder verzweigtes Alkyl mit 1 bis 4 Kohlenstoffatomen, beispielsweise Methyl, Äthyl, Isopropyl.

Bevorzugte Verbindung der Formel I sind solche, bei denen $R^1$ Äthyl, $R^2$ Alkylthio mit 3 bis 4 Kohlenstoffatomen, $R^3$ Wasserstoff oder Methyl, $R^4$ Methyl und X Schwefel bedeuten.

Die Pyrazol-5-yl(thio)(thiol)phosphor(phosphon)säureester der Formel I können durch Umsetzung von 5-Hydroxypyrazolen der Formel II in Gegenwart eines Säureakzeptors oder durch Umsetzung von Salzen, beispielsweise von Alkalimetall-, Erdalkalimetall- oder gegebenenfalls substituierten Ammoniumsalzen, dieser 5-Hydroxypyrazole mit (Thio)(thiol)phosphor(phosphon)esterhalogeniden der Formel III nach folgender Reaktionsgleichung erhalten werden:

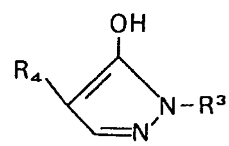

(II)

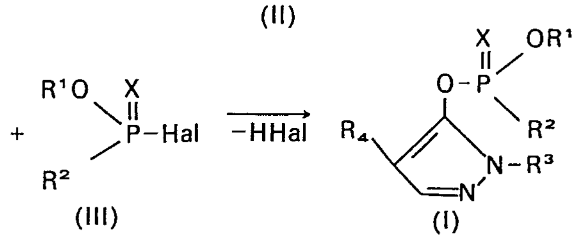


(III) (I)

In diesen Formeln haben die Substituenten die oben angegebenen Bedeutungen, und Hal steht für Halogen, vorzugsweise für Chlor.

Die Umsetzung wird zweckmässigerweise in gegenüber den Reaktionsteilnehmern inerten Lösungs- oder Verdünnungsmitteln ausgeführt. Hierfür sind beispielsweise aliphatische und aromatische, gegebenenfalls chlorierte Kohlenwasserstoffe, wie Petroläther, Benzol, Toluol, Xylol, Dichlormethan, Chloroform, Tetrachlormethan, 1,2-Dichlorethan, Chlorbenzol; Äther, wie Diäthyl- und Di-n-butyläther, Methyltert.-butyläther, Tetrahydrofuran, Dioxan; Ketone, wie Aceton, Methyläthylketon, Methylisopropylketon; ferner Nitrile, wie Acetonitril und Propionitril, geeignet. Auch Gemische dieser Lösungs- oder Verdünnungsmittel können verwendet werden.

Als Säureakzeptoren können die bei der Phosphorylierung von Hydroxyverbindungen üblichen basischen Mittel Verwendung finden. Als besonders geeignet erweisen sich Alkalimetallcarbonate oder -alkoholate, wie Natrium- und Kaliumcarbonat, -methylat und -äthylat, ferner aliphatische, aromatische und heterocyclische Amine, z.B. Triäthylamin, Dimethylamin, Piperidin, Dimethylanilin, Dimethylbenzylamin und Pyridin. In einigen Fällen ist die Verwendung von Alkyllithiumverbindungen, z.B. n-Butyllithium, vorteilhaft.

Statt in Gegenwart eines Säureakzeptors zu arbeiten, ist es ebenso gut möglich, zunächst die Salze, beispielsweise die Alkalimetall-, Erdalkalimetall- oder gegebenenfalls substituierte Ammoniumsalze der 5-Hydroxypyrazole der Formel II in Substanz herzustellen und diese anschliessend mit der Verbindung der Formel III weiter umzusetzen.

Üblicherweise setzt man die Ausgangsstoffe in äquimolarem Verhältnis ein. Ein Überschuss der einen oder anderen Reaktionskomponente kann in einigen Fällen Vorteile bringen.

Die Reaktionstemperatur ist innerhalb eines grösseren Bereiches variierbar. Im allgemeinen arbeitet man zwischen 0 und 120° C, vorzugsweise im Bereich zwischen 20 und 50° C. Da die Reaktion in einigen Fällen exotherm verläuft, kann eine Kühlung des Reaktionsgemisches von aussen zu Beginn der Reaktion zweckmässig sein.

Die Umsetzung wird üblicherweise bei Normaldruck ausgeführt.

Die 5-Hydroxypyrazole der Formel II sind zum Teil bekannt; sie lassen sich nach üblichen Kondensationsverfahren herstellen („Chem. Ber." *38*, 2104 [1905]; „Bull. Soc. Chim.", France 1967, S. 3780, 3792). Unter 5-Hydroxypyrazolen der Formel II, die als Ausgangsstoffe zur Herstellung der erfindungsgemässen Verbindungen dienen, sind auch die tautomeren Formen dieser Pyrazole zu verstehen („Bull. Soc. Chim.", France 1967, S. 3780-3794).

Die Pyrazole der Formel II lassen sich nach folgendem Reaktionsschema herstellen:

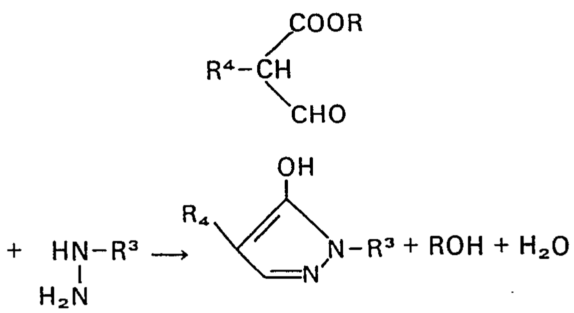

(II)

In dieser Gleichung bedeutet R einen niederen Alkylrest, die übrigen Substituenten haben die oben angegebenen Bedeutungen.

Die Umsetzung kann in Gegenwart eines Katalysators, beispielsweise einer Säure, wie Salzsäure, und gegebenenfalls in Gegenwart eines Lösungsmittels durchgeführt werden, wobei sich insbesondere Alkohole oder aromatische Kohlenwasserstoffe, wie Toluol, als Lösungsmittel eignen. Zur Vervollständigung der Umsetzung können der entstehende Alkohol und das Wasser abdestilliert werden; die Entfernung dieser Reaktionsprodukte kann durch Schleppmittel, wie Toluol, unterstützt werden.

Am Beispiel des 1-Phenyl-4-isopropyl-5-hydroxypyrazols wird die Herstellung der Pyrazole der Formel II erläutert.

Zu 28,8 g 2-Formyl-3-methylbuttersäuremethylester in 200 ml Toluol werden 21,6 g Phenylhydrazin gegeben. Die Lösung erwärmt sich dabei auf 30° C. Bei Normaldruck werden Toluol und die Nebenprodukte zum grössten Teil abdestilliert, der feste Rückstand wird aus Acetonitril umkristallisiert. Man erhält 2,53 g 1-Phenyl-4-isopropyl-5-hydroxypyrazol vom Schmelzpunkt 125-127° C.

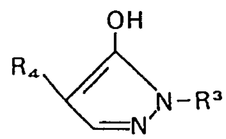

| $R^4$ | $R^3$ | Fp. (° C) |
|---|---|---|
| $CH_3$ | H | 229-233 |
| $CH_3$ | $CH_3$ | 133-135 |
| $CH_3$ | $C_6H_5$ | 160-163 |
| $CH_3$ | i-$C_3H_7$ | 177-178 |
| $C_2H_5$ | $CH_3$ | 121-124 |
| $C_2H_5$ | $C_6H_5$ | 98-100 |
| i-$C_3H_7$ | $CH_3$ | 90-92 |
| i-$C_3H_7$ | $C_6H_5$ | 125-127 |

Die zur Synthese der Verbindungen der Formel I ausserdem benötigten (Thiono)(thiol)phosphor(phosphon)säureesterhalogenide III sind aus Houben-Weyl, „Methoden der organischen Chemie", B. XII/2, S. 274 ff., Georg-Thieme-Verlag, Stuttgart, 1964, bekannt und lassen sich auf den dort beschriebenen Synthesewegen herstellen.

Die Pyrazol-5-ylphosphorsäurederivate der Formel I können folgendem Beispiel entsprechend hergestellt werden:

Zu 5,6 g 1,4-Dimethyl-5-hydroxypyrazol in 150 ml Toluol und 30 ml Methanol gibt man 9,2 ml 30%ige Lösung von Natriummethylat in Methanol. Man erhitzt 2 h lang auf Siedetemperatur und destilliert die flüchtigen Bestandteile unter vermindertem Druck ab. Dann versetzt man mit 150 ml Toluol, destilliert das Lösungsmittel ab und versetzt den festen Rückstand mit 70 ml Acetonitril. Zu dieser Suspension tropft man 7,5 g O,O-Diäthylthiophosphoresterchlorid und erhitzt das Reaktionsgemisch 7 h lang auf 50° C. Das Lösungsmittel wird unter vermindertem Druck abgezogen, der Rückstand mit 400 ml Toluol versetzt und dreimal mit je 100 ml Wasser gewaschen. Man trocknet die Toluollösung, engt sie ein und entfernt flüchtige Bestandteile bei 40° C/0,1 mbar. Man erhält O,O-Diäthyl-O-(1,4-dimethylpyrazol-5-yl)-thionophosphorsäureester als farbloses Öl; $n_D^{27}$ = 1,4918; Ausbeute: 6,5 g.

Folgende Pyrazol-5-ylphosphorsäurederivate der Formel I lassen sich entsprechend synthetisieren:

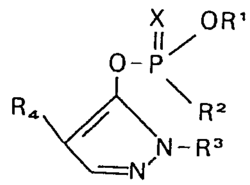

| Nr. | $R^4$ | $R^3$ | $R^2$ | $R^1$ | X | Physik. Daten |
|---|---|---|---|---|---|---|
| 1 | $CH_3$ | H | $CH_3O$ | $CH_3$ | S | Fp. 120-124°C |
| 2 | $CH_3$ | H | $C_2H_5O$ | $C_2H_5$ | S | Fp. 124-126°C |
| 3 | $CH_3$ | $CH_3$ | $CH_3O$ | $CH_3$ | S | |
| 4 | $CH_3$ | $CH_3$ | $C_2H_5O$ | $C_2H_5$ | S | $n_D^{27} = 1{,}4918$ |
| 5 | $CH_3$ | $CH_3$ | n-$C_3H_7S$ | $C_2H_5$ | S | $n_D^{23} = 1{,}5351$ |
| 6 | $CH_3$ | $CH_3$ | sek.-$C_4H_9S$ | $C_2H_5$ | S | $n_D^{23} = 1{,}5318$ |
| 7 | $CH_3$ | $CH_3$ | i-$C_4H_9S$ | $C_2H_5$ | S | $n_D^{22} = 1{,}5290$ |
| 8 | $CH_3$ | $CH_3$ | $C_2H_5O$ | $C_2H_5$ | O | $n_D^{25} = 1{,}4600$ |
| 9 | $CH_3$ | $CH_3$ | n-$C_3H_7S$ | $C_2H_5$ | O | $n_D^{23} = 1{,}4907$ |
| 10 | $CH_3$ | $CH_3$ | sek.-$C_4H_9S$ | $C_2H_5$ | O | $n_D^{23} = 1{,}4910$ |
| 11 | $CH_3$ | $CH_3$ | i-$C_4H_9S$ | $C_2H_5$ | O | |
| 12 | $CH_3$ | $CH_3$ | $C_2H_5O-(CH_2)_2S$ | $C_2H_5$ | O | $n_D^{23} = 1{,}4932$ |
| 13 | $CH_3$ | $CH_3$ | $CH_3O-(CH_2)_2S$ | $C_2H_5$ | O | |
| 14 | $CH_3$ | $CH_3$ | $Cl-(CH_2)_3S$ | $C_2H_5$ | O | |
| 15 | $CH_3$ | $C_6H_5$ | $CH_3O$ | $CH_3$ | S | |
| 16 | $CH_3$ | $C_6H_5$ | $C_2H_5O$ | $C_2H_5$ | O | |
| 17 | $CH_3$ | $C_6H_5$ | $C_2H_5O$ | $C_2H_5$ | S | $n_D^{24} = 1{,}5454$ |
| 18 | $CH_3$ | $C_6H_5$ | n-$C_3H_7S$ | $C_2H_5$ | S | $n_D^{25} = 1{,}5733$ |
| 19 | $CH_3$ | $C_6H_5$ | sek.-$C_4H_9S$ | $C_2H_5$ | S | $n_D^{22} = 1{,}5616$ |
| 20 | $CH_3$ | $C_6H_5$ | i-$C_4H_9S$ | $C_2H_5$ | S | $n_D^{22} = 1{,}5662$ |
| 21 | $CH_3$ | $C_6H_5$ | n-$C_4H_9S$ | $C_2H_5$ | S | |
| 22 | $CH_3$ | $C_6H_5$ | $C_2H_5O$ | $C_2H_5$ | O | |
| 23 | $CH_3$ | $C_6H_5$ | n-$C_3H_7S$ | $C_2H_5$ | O | $n_D^{22} = 1{,}5468$ |
| 24 | $CH_3$ | $C_6H_5$ | sek.-$C_4H_9S$ | $C_2H_5$ | O | $n_D^{22} = 1{,}5453$ |
| 25 | $CH_3$ | $C_6H_5$ | i-$C_4H_9S$ | $C_2H_5$ | O | |
| 26 | $CH_3$ | $C_6H_5$ | $C_2H_5O-(CH_2)_2S$ | $C_2H_5$ | O | $n_D^{23} = 1{,}5417$ |
| 27 | $CH_3$ | $C_6H_5$ | $CH_3O-(CH_2)_2S$ | $C_2H_5$ | O | $n_D^{22} = 1{,}5519$ |
| 28 | $CH_3$ | i-$C_3H_7$ | $C_2H_5O$ | $C_2H_5$ | S | |
| 29 | $CH_3$ | i-$C_3H_7$ | sek.-$C_4H_9S$ | $C_2H_5$ | S | |
| 30 | $C_2H_5$ | $CH_3$ | $C_2H_5O$ | $C_2H_5$ | S | $n_D^{22} = 1{,}4932$ |
| 31 | $C_2H_5$ | $CH_3$ | n-$C_3H_7S$ | $C_2H_5$ | S | $n_D^{22} = 1{,}5309$ |
| 32 | $C_2H_5$ | $CH_3$ | sek.-$C_4H_9S$ | $C_2H_5$ | S | |
| 33 | $C_2H_5$ | $CH_3$ | i-$C_4H_9S$ | $C_2H_5$ | S | $n_D^{23} = 1{,}5204$ |
| 34 | $C_2H_5$ | $CH_3$ | n-$C_3H_7S$ | $C_2H_5$ | O | $n_D^{22} = 1{,}4953$ |
| 35 | $C_2H_5$ | $CH_3$ | sek.-$C_4H_9S$ | $C_2H_5$ | O | $n_D^{22} = 1{,}4927$ |
| 36 | $C_2H_5$ | $CH_3$ | i-$C_4H_9S$ | $C_2H_5$ | O | $n_D^{24} = 1{,}4900$ |
| 37 | $C_2H_5$ | $CH_3$ | $CH_3O-(CH_2)_2S$ | $C_2H_5$ | O | |
| 38 | $C_2H_5$ | $CH_3$ | $C_2H_5O-(CH_2)_2S$ | $C_2H_5$ | O | $n_D^{24} = 1{,}4910$ |
| 39 | $C_2H_5$ | $C_6H_5$ | $C_2H_5O$ | $C_2H_5$ | S | Öl |
| 40 | $C_2H_5$ | $C_6H_5$ | n-$C_3H_7S$ | $C_2H_5$ | S | $n_D^{24} = 1{,}5695$ |
| 41 | $C_2H_5$ | $C_6H_5$ | sek.-$C_4H_9S$ | $C_2H_5$ | S | |
| 42 | $C_2H_5$ | $C_6H_5$ | i-$C_4H_9S$ | $C_2H_5$ | S | $n_D^{24} = 1{,}5607$ |
| 43 | $C_2H_5$ | $C_6H_5$ | $C_2H_5O$ | $C_2H_5$ | O | $n_D^{26} = 1{,}5220$ |
| 44 | $C_2H_5$ | $C_6H_5$ | n-$C_3H_7S$ | $C_2H_5$ | O | $n_D^{23} = 1{,}5420$ |
| 45 | $C_2H_5$ | $C_6H_5$ | sek.-$C_4H_9S$ | $C_2H_5$ | O | $n_D^{25} = 1{,}5382$ |
| 46 | $C_2H_5$ | $C_6H_5$ | i-$C_4H_9S$ | $C_2H_5$ | O | $n_D^{25} = 1{,}5390$ |

| Nr. | $R^4$ | $R^3$ | $R^2$ | $R^1$ | X | Physik. Daten |
|---|---|---|---|---|---|---|
| 47 | $C_2H_5$ | $C_6H_5$ | $CH_3O-(CH_2)_2S$ | $C_2H_5$ | O | |
| 48 | $C_2H_5$ | $C_6H_5$ | $C_2H_5O-(CH_2)_2S$ | $C_2H_5$ | O | $n_D^{24}$ = 1,5391 |
| 49 | i-$C_3H_7$ | H | $C_2H_5O$ | $C_2H_5$ | S | |
| 50 | i-$C_3H_7$ | $CH_3$ | $CH_3O$ | $CH_3$ | S | |
| 51 | i-$C_3H_7$ | $CH_3$ | $C_2H_5O$ | $C_2H_5$ | S | $n_D^{22}$ = 1,4890 |
| 52 | i-$C_3H_7$ | $CH_3$ | n-$C_3H_7S$ | $C_2H_5$ | S | $n_D^{25}$ = 1,5260 |
| 53 | i-$C_3H_7$ | $CH_3$ | sek.-$C_4H_9S$ | $C_2H_5$ | S | $n_D^{26}$ = 1,5246 |
| 54 | i-$C_3H_7$ | $CH_3$ | i-$C_4H_9S$ | $C_2H_5$ | S | $n_D^{26}$ = 1,5200 |
| 55 | i-$C_3H_7$ | $CH_3$ | i-$C_3H_7S$ | $C_2H_5$ | S | |
| 56 | i-$C_3H_7$ | $CH_3$ | $C_2H_5O$ | $C_2H_5$ | O | |
| 57 | i-$C_3H_7$ | $CH_3$ | n-$C_3H_7S$ | $C_2H_5$ | O | $n_D^{22}$ = 1,4930 |
| 58 | i-$C_3H_7$ | $CH_3$ | sek.-$C_4H_9S$ | $C_2H_5$ | O | $n_D^{27}$ = 1,4895 |
| 59 | i-$C_3H_7$ | $CH_3$ | i-$C_4H_9S$ | $C_2H_5$ | O | |
| 60 | i-$C_3H_7$ | $CH_3$ | $C_2H_5O-(CH_2)_2S$ | $C_2H_5$ | O | $n_D^{23}$ = 1,4906 |
| 61 | i-$C_3H_7$ | $CH_3$ | $CH_3O-(CH_2)_2S$ | $C_2H_5$ | O | |
| 62 | i-$C_3H_7$ | $C_6H_5$ | $CH_3O$ | $CH_3$ | S | |
| 63 | i-$C_3H_7$ | $C_6H_5$ | $C_2H_5O$ | $C_2H_5$ | S | $n_D^{23}$ = 1,5356 |
| 64 | i-$C_3H_7$ | $C_6H_5$ | n-$C_3H_7S$ | $C_2H_5$ | S | $n_D^{29}$ = 1,5560 |
| 65 | i-$C_3H_7$ | $C_6H_5$ | sek.-$C_4H_9S$ | $C_2H_5$ | S | $n_D^{22}$ = 1,5535 |
| 66 | i-$C_3H_7$ | $C_6H_5$ | i-$C_4H_9S$ | $C_2H_5$ | S | $n_D^{27}$ = 1,5535 |
| 67 | i-$C_3H_7$ | $C_6H_5$ | i-$C_4H_9S$ | $C_2H_5$ | S | |
| 68 | i-$C_3H_7$ | $C_6H_5$ | n-$C_3H_7S$ | $C_2H_5$ | O | $n_D^{27}$ = 1,5360 |
| 69 | i-$C_3H_7$ | $C_6H_5$ | sek.-$C_4H_9S$ | $C_2H_5$ | O | $n_D^{23}$ = 1,5360 |
| 70 | i-$C_3H_7$ | $C_6H_5$ | i-$C_4H_9S$ | $C_2H_5$ | O | |
| 71 | i-$C_3H_7$ | $C_6H_5$ | $C_2H_5O-(CH_2)_2S$ | $C_2H_5$ | O | $n_D^{25}$ = 1,5313 |
| 72 | i-$C_3H_7$ | $C_6H_5$ | $CH_3O-(CH_2)_2S$ | $C_2H_5$ | O | |
| 73 | i-$C_3H_7$ | $C_6H_5$ | $CH_3$ | $C_2H_5$ | O | |
| 74 | i-$C_3H_7$ | $C_6H_5$ | $C_6H_5$ | $C_2H_5$ | O | |

Die erfindungsgemässen Pyrazol-5-yl(thio)-(thiol)phosphor(phosphon)säureester der Formel I sind geeignet, Schädlinge, insbesondere aus der Klasse der Insekten und Spinnentiere, wirksam zu bekämpfen. Sie können im Pflanzenschutz sowie auf dem Hygiene-, Vorratsschutz- und Veterinärsektor eingesetzt werden.

Zu diesen Schädlingen gehören Insekten aus der Ordnung der Schmetterlinge *(Lepidoptera)*, beispielsweise *Plutella maculipennis* (Kohlschabe), *Leucoptera coffeella* (Kaffeemotte), *Hyponomeuta malinellus* (Apfelbaumgespinstmotte), *Argyresthia conjugella* (Apfelmotte), *Sitotroga cerealella* (Getreidemotte), *Phthorimaea operculella* (Kartoffelmotte), *Capua reticulana* (Apfelschalenwickler), *Sparganothis pilleriana* (Springwurm), *Cacoecia murinana* (Tannentriebwickler), *Tortrix viridana* (Eichenwickler), *Clysia ambiguella* (Heu- und Sauerwurm), *Evetria buoliana* (Kieferntriebwickler), *Polychrosis botrana* (Bekreuzter Traubenwickler), *Cydia pomonella* (Obstmade), *Laspeyresia molesta* (Pfirsichtriebbohrer), *Laspeyresia funebrana* (Pflaumenwickler), *Ostrinia nubilalis* (Maiszünsler), *Loxostege sticticalis* (Rübenzünsler), *Ephestia kuehniella* (Mehlmotte), *Chilo suppressalis* (Reisstengelbohrer), *Galleria mellonella* (Wachsmotte), *Malacosoma neustria* (Ringelspinner), *Dendrolimus pini* (Kiefernspinner), *Thaumatopoea pityocampa* (Pinienprozessionsspinner), *Phalera bucephala* (Mondfleck), *Cheimatobia brumata* (Kleiner Frostspanner), *Hibernia defoliaria* (Grosser Frostspanner), *Bupalus piniarius* (Kiefernspanner), *Hyphantria cunea* (Weisser Bärenspinner), *Agrotis segetum* (Wintersaateule), *Agrotis ypsilon* (Ypsiloneule), *Barathra brassicae* (Kohleule), *Cirphis unipuncta* (Heerwurm), *Prodenia litura* (Baumwollraupe), *Laphygma exigua* (Rüben-Heerwurm), *Panolis flammea* (Forleule), *Earias insulana* (Baumwollkapselwurm), *Plusia gamma* (Gammaeule), *Alabama argillacea* (Baumwollblattwurm), *Lymantria dispar* (Schwammspinner), *Lymantria monacha* (Nonne), *Pieris brassicae* (Kohlweissling), *Aporia crataegi* (Baumweissling);

aus der Ordnung der Käfer *(Coleoptera)*, beispielsweise *Blitophaga undata* (Schwarzer Rübenaaskäfer), *Melanotus communis* (Drahtwurm), *Limonius californicus* (Drahtwurm), *Agriotes lineatus* (Saatschnellkäfer), *Agriotes obscurus* (Humusschnellkäfer), *Agrilus sinuatus* (Birnbaum-Prachtkäfer), *Meligethes aeneus* (Rapsglanzkäfer), *Atomaria linearis* (Moosknopfkäfer), *Epilachna varivestis* (Mexikanischer Bohnenkäfer), *Phyllopertha horticola* (Junikäfer), *Popillia*

*japonica* (Japankäfer), *Melolontha melolontha* (Feldmaikäfer), *Melolontha hippocastani* (Waldmaikäfer), *Amphimallus solstitialis* (Brachkäfer), *Crioceris asparagi* (Spargelhähnchen), *Lema melanopus* (Getreidehähnchen), *Leptinotarsa decemlineata* (Kartoffelkäfer), *Phaedon cochleariae* (Meerrettich-Blattkäfer), *Phyllotreta nemorum* (Kohlerdfloh), *Chaetocnema tibialis* (Rübenflohkäfer), *Phylloides chrysocephala* (Raps-Flohkäfer), *Diabrotica 12-punctata* (Südlicher Maiswurzelwurm), *Cassida nebulosa* (Nebliger Schildkäfer), *Bruchus lentis* (Linsenkäfer), *Bruchus rufimanus* (Pferdebohnenkäfer), *Bruchus pisorum* (Erbsenkäfer), *Sitona lineatus* (Linierter Blattrandkäfer), *Otiorrhynchus sulcatus* (Gefurchter Lappenrüssler), *Otiorrhynchus ovatus* (Erdbeerwurzelrüssler), *Hylobies abietis* (Grosser Brauner Rüsselkäfer), *Byctiscus betulae* (Rebenstecher), *Anthonomus pomorum* (Apfelblütenstecher), *Anthonomus grandis* (Kapselkäfer), *Ceuthorrhynchus assimilis* (Kohlschotenrüssler), *Ceuthorrhynchus napi* (Grosser Kohltriebrüssler), *Sitophilus granaria* (Kornkäfer), *Anisandrus dispar* (Ungleicher Holzborkenkäfer), *Ips typographus* (Bruchdrucker), *Blastophagus piniperda* (Gefurchter Waldgärtner);

aus der Ordnung der Zweiflügler *(Diptera)*, beispielsweise *Lycoria pectoralis, Mayetiola destructor* (Hessenfliege), *Dasineura brassicae* (Kohlschoten-Gallmücke), *Contarinia tritici* (Gelbe Weizen-Gallmücke), *Haplodiplosis equestris* (Sattelmücke), *Tipula oleracea* (Kohlschnake), *Dacus cucurbitae* (Melonenfliege), *Dacus oleae* (Olivenfliege), *Ceratitis capitata* (Mittelmeerfruchtfliege), *Rhagoletis cerasi* (Kirschfruchtfliege), *Rhagoletis pomonella* (Apfelmade), *Anastrepha ludens* (Mexikanische Fruchtfliege), *Oscinella frit* (Fritfliege), *Phorbia coarctata* (Brachfliege), *Phorbia antiqua* (Zwiebelfliege), *Phorbia brassicae* (Kleine Kohlfliege), *Pegomya hyoscyami* (Rübenfliege), *Anopheles maculiphennis, Culex pipiens, Aedes aegypti* (Gelbfiebermücke), *Aedes vexans, Tabanus bovinus* (Rinderbremse), *Tipula paludosa* (Wiesenschnake), *Musca domestica* (Stubenfliege), *Fannia canicularis* (Kleine Stubenfliege), *Muscina stabulans, Glossina morsitans* (Tsetse-Fliege), *Oestrus ovis, Chrysomya macellaria, Chrysomya hominivorax, Lucilia cuprina, Lucilia sericata, Hypoderma lineata*;

aus der Ordnung der Hauftflügler *(Hymenoptera)*, beispielsweise *Athalia rosae* (Rübenblattwespe), *Hoplocampa minuta* (Pflaumensägewespe), *Monomorium pharaonis* (Pharaoameise), *Solenopsis geminata* (Feuerameise), *Atta sexdens* (Blattschneiderameise);

aus der Ordnung der Wanzen *(Heteroptera)*, beispielsweise *Nezara viridula* (Grüne Reiswanze), *Eurygaster integriceps* (Asiatische Getreidewanze), *Blissus leucopterus* (Chinch bug), *Dysdercus cingulatus* (Kapok-Wanze), *Dysdercus intermedius* (Baumwollwanze), *Piesma quadrata* (Rübenwanze), *Lygus pratensis* (Gemeine Wiesenwanze);

aus der Ordnung der Pflanzensauger *(Homoptera)*, beispielsweise *Perkinsiella saccharicida* (Zuckerrohrzikade), *Nilaparvata lugens* (Braune Zikade), *Empoasca fabae* (Kartoffelzikade), *Psylla mali* (Apfelblattsauger), *Psylla piri* (Birnblattsauger), *Trialeurodes vaporariorum* (Weisse Fliege), *Aphis fabae* (Schwarze Bohnenlaus), *Aphis pomi* (Grüne Apfellaus), *Aphis sambuci* (Holunderblattlaus), *Aphidula nastrutii* (Kreuzdornblattlaus), *Cerosipha gossypii* (Gurkenblattlaus), *Sappaphis mali* (Rosige Apfellaus), *Sappaphis mala* (Mehlige Birnblattlaus), *Dysphis radicola* (Mehlige Apfelfalterlaus), *Brachycaudus cardui* (Grosse Pflaumenblattlaus), *Brevicoryne brassicae* (Kohlblattlaus), *Phorodon humili* (Hopfenblattlaus), *Rhopalomyzus ascalonicus* (Zwiebellaus), *Myzodes persicae* (Grüne Pfirsichlaus), *Myzus cerasi* (Schwarze Sauerkirschenlaus), *Dysaulacorthum pseudosolani* (Gefleckte Kartoffellaus), *Acyrthosiphon onobrychis* (Grüne Erbsenlaus), *Macrosiphon rasae* (Grosse Rosenblattlaus), *Megoura viciae* (Wickenlaus), *Schizoneura lanuginosa* (Birnenblattlaus), *Pemphigus bursarius* (Salatwurzellaus), *Dreyfusia nordmannianae* (Tannentrieblaus), *Dreyfusia piceae* (Weisstannenstammlaus), *Adelges laricis* (Rote Fichtengallenlaus), *Viteus vitifolii* (Reblaus);

aus der Ordnung der Termiten *(Isoptera)*, beispielsweise *Reticulitermes lucifugus, Calotermes flavicollis, Leucotermes flavipes, Termes natalensis*, und

aus der Ordnung der Geradflüger *(Orthoptera)*, beispielsweise *Forficula auricularia* (Gemeiner Ohrwurm), *Acheta domestica* (Heimchen), *Gryllotalpa gryllotalpa* (Maulwurfsgrille), *Tachycines asynamorus* (Gewächshausschrecke), *Locusta migratoria* (Wanderheuschrecke), *Stauronotus maroccanus* (Marokkanische Wanderheuschrekke), *Schistocerca peregrina* (Wanderheuschrekke), *Nomadacris septemfasciata* (Wanderheuschrecke), *Melanoplus spretus* (Felsengebirgsheuschrecke), *Melano plus femur-rubrum* (Rotbeinige Heuschrecke), *Blatta orientalis* (Küchenschabe), *Blattella germanica* (Deutsche Schabe), *Periplaneta americana* (Amerikanische Schabe), *Blabera gigantea* (Riesenschabe).

Zur Klasse der Spinnentiere *(Arachnoidea)* gehören Milben und Zecken *(Acarina)*, beispielsweise *Ixodes ricinus* (Holzbock), *Ornithodorus moubata, Amblyomma americanum, Dermacentor silvarum, Boophilus microplus, Tetranychus telarius, Tetranychus atlanticus, Tetranychus pacificus, Paratetranychus pilosus, Bryobia praetiosa.*

Die Wirkstoffe können als solche, in form ihrer formulierungen oder den daraus bereiteten Anwendungsmöglichkeiten, z.B. in form von direkt versprühbaren Lösungen, Pulvern, Suspensionen oder Dispersionen, Emulsionen, Öldispersionen, Pasten, Stäubemitteln, Streumitteln, Granulaten durch Versprühen, Vernebeln, Verstäuben, Verstreuen oder Giessen angewendet werden. Die Anwendungsformen richten sich ganz nach den Verwendungszwecken; sie sollten in jedem Fall möglichst die feinste Verteilung der erfindungsgemässen Wirkstoffe gewährleisten.

Zur Herstellung von direkt versprühbaren Lösungen, Emulsionen, Pasten oder Öldispersionen

kommen Mineralölfraktionen von mittlerem bis bohem Siedepunkt, wie Kerosin oder Dieselöl, ferner Kohlenteeröle sowie Öle pflanzlichen oder tierischen Ursprungs, aliphatische, cyclische und aromatische Kohlenwasserstoffe, z.B. Benzol, Toluol, Xylol, Paraffin, Tetrahydronaphthalin, alkylierte Naphthaline oder deren Derivate, z.B. Methanol, Äthanol, Propanol, Butanol, Chloroform, Tetrachlorkohlenstoff, Cyclohexanol, Cyclohexanon, Chlorbenzol, Isophoron, stark polare Lösungsmitteln, z.B. Dimethylformamid, Dimethylsulfoxid, N-Methylpyrrolidon, Wasser, in Betracht.

Wässerige Anwendungsformen können aus Emulsionskonzentraten, Pasten oder netzbaren Pulvern (Spiritzpulvern, Öldispersionen) durch Zusatz von Wasser bereitet werden. Zur Herstellung von Emulsionen, Pasten oder Öldispersionen können die Substanzen als solche oder in einem Öl oder Lösungsmittel gelöst, mittels Netz-, Haft-, Dispergier- oder Emulgiermittel in Wasser homogenisiert werden. Es können aber auch aus wirksamer Substanz Netz-, Haft-, Dispergier- oder Emulgiermittel und eventuell Lösungsmittel oder Öl bestehende Konzentrate hergestellt werden, die zur Verdünnung mit Wasser geeignet sind.

Als oberflächenaktive Stoffe kommen Alkali-, Erdalkali-, Ammoniumsalze von Ligninsulfonsäure, Naphthalinsulfonsäure, Phenolsulfonsäure, Alkylarylsulfonate, Alkylsulfate, Alkylsulfonate, Alkali- und Erdalkalisalze der Dibutylnaphthalinsulfonsäure, Lauryläthersulfat, Fettalkoholsulfate, fettsaure Alkali- und Erdalkalisalze, Salze sulfatierter Hexadecanole, Heptadecanole, Octadecanole, Salze von sulfatiertem Fettalkoholglykoläther, Kondensationsprodukte von sulfoniertem Naphthalin und Naphthalinderivaten mit Formaldehyd, Kondensationsprodukte des Naphthalins bzw. der Naphthalinsulfonsäuren mit Phenol und Formaldehyd, Polyoxyäthylenoctylphenoläther, äthoxyliertes Isooctylphenol, Octylphenol, Nonylphenon, Alkylphenolpolyglykoläther, Tributylphenylpolyglykoläther, Alkylarylpolyätheralkohole, Isotridecylalkohol, Fettalkoholäthylenoxidkondensate, äthoxyliertes Rizinusöl, Polyoxyäthylenalkyläther, äthoxyliertes Polyoxypropylen, Laurylalkoholpolyglykolätheracetal, Sorbitester, Lignin, Sulfitablaugen und Methylcellulose in Betracht.

Pulver-, Streu- und Stäubemittel können durch Mischen oder gemeinsames Vermahlen der wirksamen Substanzen mit einem festen Trägerstoff hergestellt werden.

Granulate, z.B. Umhüllungs-, Imprägnierungs- und Homogengranulate, können durch Bindung der Wirkstoffe an feste Trägerstoffe hergestellt werden. Feste Trägerstoffe sind z.B. Mineralerden, wie Silikagel, Kieselsäuren, Kieselgele, Silikate, Talkum, Kaolin, Attaclay, Kalkstein, Kalk, Kreide, Bolus, Löss, Ton, Dolomit, Diatomeenerde, Calcium- und Magnesiumsulfat, Magnesiumoxid, gemahlene Kunststoffe, Düngemittel, wie z.B. Ammoniumsulfat, Ammoniumphosphat, Ammoniumnitrat, Harnstoffe und pflanzliche Produkte, wie Getreidemehl, Baumrinden-, Holz- und Nusschalenmehl, Cellulosepulver und andere feste Trägerstoffe.

Beispiele für Formulierungen sind:

I. 3 Gew.-Teile des Wirkstoffs Nr. 1 werden mit 97 Gew.-Teilen feinteiligem Kaolin innig vermischt. Man erhält auf diese Weise ein Stäubemittel, das 3 Gew.-% des Wirkstoffs enthält.

II. 30 Gew.-Teile des Wirkstoffs Nr. 5 werden mit einer Mischung aus 92 Gew.-Teilen pulverförmigem Kieselsäuregel und 8 Gew.-Teilen Paraffinöl, das auf die Oberfläche dieses Kieselsäuregels gesprüht wurde, innig vermischt. Man erhält auf diese Weise eine Aufbereitung des Wirkstoffs mit guter Haftfähigkeit.

III. 20 Gew.-Teile des Wirkstoffs Nr. 20 werden in einer Mischung gelöst, die aus 80 Gew.-Teilen Xylol, 10 Gew.-Teilen des Anlagerungsproduktes von 8 bis 10 mol Äthylenoxid an 1 mol Ölsäure-N-monoäthanolamid, 5 Gew.-Teilen Calciumsalz der Dodecylbenzolsulfonsäure und 5 Gew.-Teilen des Anlagerungsproduktes von 40 mol Äthylenoxid an 1 mol Rizinusöl besteht. Durch Ausgiessen und feines Verteilen der Lösung in 100 000 Gew.-Teilen Wasser erhält man eine Wässerige Dispersion, die 0,02 Gew.-% des Wirkstoffs enthält.

IV. 20 Gew.-Teile des wirkstoffs Nr. 54 werden in einer Mischung gelöst, die aus 40 Gew.-Teilen Cyclohexanon, 30 Gew.-Teilen Isobutanol, 20 Gew.-Teilen des Anlagerungsproduktes von 7 mol Äthylenoxid an 1 mol Isooctylphenol und 10 Gew.-Teilen des Anlagerungsproduktes von 40 mol Äthylenoxid an 1 mol Rizinusöl besteht. Durch Eingiessen und feines Verteilen der Lösung in 100 000 Gew.-Teilen Wasser erhält man eine wässerige Dispersion, die 0,02 Gew.-% des Wirkstoffs enthält.

Die Formulierungen enthalten im allgemeinen zwischen 0,1 und 95 Gew.-% Wirkstoff, vorzugsweise zwischen 0,5 und 90 Gew.-%. Die Wirkstoffe werden in Form ihrer Formulierungen oder in Form der aus diesen Formulierungen bereiteten anwendungsfertigen Zubereitungen angewendet. Der Wirkstoffgehalt in diesen Anwendungsformen kann in weiten Bereichen variieren. Im allgemeinen liegt er zwischen 0,0001 und 10%, vorzugsweise zwischen 0,01 und 5%.

Die Wirkstoffe können auch mit gutem Erfolg im Ultra-Low-Volume-Verfahren (ULV) verwendet werden, wobei es möglich ist, Formulierungen mit mehr als 95 Gew.-% Wirkstoff oder sogar den Wirkstoff ohne Zusätze auszubringen.

Die Aufwandmenge an Wirkstoff beträgt unter Freilandbedingungen 0,2 bis 10 kg/ha, vorzugsweise 0,5 bis 2,0 kg/ha.

Zu den Wirkstoffen können Öle verschiedenen Typs, Herbizide, Fungizide, andere Insektizide, Bakterizide, gegebenenfalls auch erst unmittelbar vor der Anwendung (Tankmix), zugesetzt werden. Diese Mittel können zu den erfindungsgemässen Mitteln im Gewichtsverhältnis 1:10 bis 10:1 zugemischt werden.

Beispielsweise können folgende Mittel zugemischt werden:

1,2-Dibrom-3-chlorpropan,

1,3-Dichlorpropen,

1,3-Dichlorpropen + 1,2-Dichlorpropan,
1,2-Dibromäthan,
2-sek.-Butylphenyl-N-methylcarbamat,
o-Chlorphenyl-N-methylcarbamat,
3-Isopropyl-5-methylphenyl-N-methylcarbamat,
o-Isopropoxyphenyl-N-methylcarbamat,
3,5-Dimethyl-4-methylmercaptophenyl-N-methylcarbamat,
4-Dimethylamino-3,5-xylyl-N-methylcarbamat,
2-(1,3-Dioxolan-2-yl)phenyl-N-methylcarbamat,
1-Naphthyl-N-methylcarbamat,
2,3-Dihydro-2,2-dimethylbenzofuran-7-yl-N-methylcarbamat,
2,2-Dimethyl-1,3-benzodioxol-4-yl-N-methylcarbamat,
2-Dimethylamino-5,6-dimethyl-4-pyrimidinyl-dimethylcarbamat,
2-Methyl-2-(methylthio)propionaldehyd-O-(methylcarbamoyl)oxim,
S-Methyl-N-[(methylcarbamoyl)oxy]thioacetimidat,
Methyl-N',N'-dimethyl-N-[(methylcarbamoyl)-oxy]-1-thiooxamidat,
N-(2-Methyl-4-chlorphenyl)-N',N'-dimethylformamidin,
Tetrachlorthiophen,
1-(2,6-Difluorbenzoyl)-3-(4-chlorphenyl)-harnstoff,
O,O-Dimethyl-O-(p-nitrophenyl)phosphorthioat,
O,O-Diäthyl-O-(p-nitrophenyl)phosphorthioat,
O-Äthyl-O-(p-nitrophenyl)phenylphosphonothioat,
O,O-Dimethyl-O-(3-methyl-4-nitrophenyl)-phosphorthioat,
O,O-Diäthyl-O-(2,4-dichlorphenyl)phosphorthioat,
O-Äthyl-O-(2,4-dichlorphenyl)phenylphosphonothioat,
O,O-Dimethyl-O-(2,4,5-trichlorphenyl)-phosphorthioat,
O-Äthyl-O-(2,4,5-trichlorphenyl)äthylphosphonothioat,
O,O-Dimethyl-O-(4-brom-2,5-dichlorphenyl)-phosphorthioat,
O,O-Dimethyl-O-(2,5-dichlor-4-jodphenyl)-phosphorthioat,
O,O-Dimethyl-O-(3-methyl-4-methylthiophenyl)phosphorthioat,
O-Äthyl-O-(3-methyl-4-methylthiophenyl)isopropylphosphoramidat,
O,O-Diäthyl-O-[(p-methylsulfinyl)phenyl]-phosphorthioat,
O-Äthyl-S-phenylethylphosphonodithioat,
O,O-Diäthyl-[2-chlor-1-(2,4-dichlorphenyl)-vinyl]phosphat,
O,O-Dimethyl-[2-chlor-1-(2,4,5-trichlorphenyl)]vinylphosphat,
O,O-Dimethyl-S-(1'phenyl)äthylacetatphosphordithioat,
Bis(dimethylamino)fluorphosphinoxid,
Octamethylpyrophosphoramid,
O,O,O,O-Tetraäthyldithiopyrophosphat,
S-Chlormethyl-O,O-diäthylphosphordithioat,
O-Ethyl-S,S-dipropylphosphordithioat,
O,O-Dimethyl-O-2,2-dichlorvinylphosphat,
O,O-Dimethyl-1,2-dibrom-2,2-dichlorethylphosphat,
O,O-Dimethyl-2,2,2-trichlor-1-hydroxyethylphosphonat,
O,O-Dimethyl-S-[1,2-biscarbäthoxyethyl-(1)]-phosphordithioat,
O,O-Dimethyl-O-(1-methyl-2-carbmethoxyvinyl)phosphat,
O,O-Dimethyl-S-(N-methylcarbamoylmethyl)-phosphordithioat,
O,O-Dimethyl-S-(N-methylcarbamoylmethyl)-phosphorthioat,
O,O-Dimethyl-S-(N-methoxyethylcarbamoylmethyl)phosphordithioat,
O,O-Dimethyl-S-(N-formyl-N-methylcarbamoylmethylphosphordithioat,
O,O-Dimethyl-O-[1-methyl-2-(methylcarbamoyl)vinyl]phosphat,
O,O-Dimethyl-O-[(1-methyl-2-dimethylcarbamoyl)vinyl]phosphat,
O,O-Dimethyl-O-[(1-methyl-2-chlor-2-diäthylcarbamoyl)vinyl]phosphat,
O,O-Diäthyl-S-(ethyl-thio-methyl)-phosphordithioat,
O,O-Diäthyl-S-[(p-chlorphenylthio)methyl]-phosphordithioat,
O,O-Dimethyl-S-(2-ethylthioethyl)phosphorthioat,
O,O-Dimethyl-S-(2-ethylthioethyl)phosphordithioat,
O,O-Dimethyl-S-(2-ethylsulfinylethyl)phosphorthioat,
O,O-Diäthyl-S-(2-ethylthioethyl)phosphordithioat,
O,O-Diäthyl-S-(2-ethylsulfinylethyl)-phosphorthioat,
O,O-Diäthylthiophosphoryliminophenylacetonitril,
O,O-Diäthyl-S-(2-chlor-1-phthalimidoethyl)-phosphordithioat,
O,O-Diäthyl-S-[6-chlorbenzoxazolon-(2)-yl-(3)]-methyldithiophosphat,
O,O-Dimethyl-S-[2-methoxy-1,3,4-thiadiazol-5-[4H]-onyl-(4)-methyl]phosphordithioat,
O,O-Diäthyl-O-[3,5,6-trichlorpyridyl-(2)]-phosphorthioat,
O,O-Diäthyl-O-(2-pyrazinyl)phosphorthioat,
O,O-Diäthyl-O-[2-isopropyl-4-methylpyrimidinyl-(6)]-phosphorthioat,
O,O-Diäthyl-O-[2-(diäthylamino)-6-methyl-4-pyrimidinyl]thionophosphat,
O,O-Dimethyl-S-(4-oxo-1,2,3-benzotriazin-3-[4H]-ylmethyl)phosphordithioat,
O,O-Dimethyl-S-[(4,6-diamino-1,3,5-triazin-2-yl)methyl]phosphordithioat,
O,O-Diäthyl-(1-phenyl-1,2,4-triazol-3-yl)-thionophosphat,
O,S-Dimethylphosphoramidothioat,
O,S-Dimethyl-N-acetylphosphoramidothioat,
γ-Hexachlorcyclohexan,
1,1-Di-(p-methoxyphenyl)-2,2,2-trichloräthan,
6,7,8,9,10,10-Hexachloro-1,5,5a,6,9,9a-hexahydro-6,9-methano-2,4,3-benzodioxathiepin-3-oxid,

Pyrethine,
DL-2-Allyl-3-methylcyclopenten-(2)-on-(1)-yl-(4)-DL-cis,transchrysanthemat,
5-Benzylfuryl-(3)-methyl-DL-cis,trans-chrysanthemat,
3-Phenoxybenzyl-(±)-cis,trans-2,2-dimethyl-3-(2,2-dichlorvinyl)cyclopropancarboxylat,
α-Cyano-3-phenoxybenzyl-(±)-cis,trans-2,2-dimethyl-3-(2,2-dichlorvinyl)cyclopropancarboxylat,
(s)-α-Cyano-3-phenoxybenzylcis-(1R,3R)-2,2-dimethyl-3-(2,2-dibromvinyl)cyclopropancarboxylat,
3,4,5,6-Tetrahydrophthalimidoäthyl-DL-cis,transchrysanthemat,
2-Methyl-5-(2-propinyl)-3-furylmethyl-chrysanthemat,
(α-Cyano-3-phenoxybenzyl)-α-isopropyl-4-chlorphenylacetat.

Die folgenden Beispiele belegen die biologische Wirkung der neuen Verbindungen.

Die Numerierung der Wirkstoffe entspricht der der tabellarischen Auflistung.

*Beispiel A:*

*Kontaktwirkung auf Schaben* (Blatta orientalis)

Der Boden eines 1-l-Glases wird mit der acetonischen Lösung des Wirkstoffes behandelt. Nach dem Verdunsten des Lösungsmittels setzt man je Glas 5 adulte Schaben. Die Mortalität wird nach 48 h bestimmt.

In diesem Test zeigen die Wirkstoffe Nrn. 4, 5, 6, 7, 9, 10, 18, 19, 20, 23, 24, 26, 51, 52, 53, 54, 57 und 58 eine gute Wirkung.

*Beispiel B:*

*Kontaktwirkung auf Moskito-Larven* (Aedes aegypti)

200 ml Leitungswasser werden mit der Wirkstoffaubereitung versetzt und darauf mit 30 bis 40 Moskito-Larven im 4. Larvenstadium besetzt. Die Versuchstemperatur beträgt 20°C. Nach 25 h wird die Wirkung ermittelt.

Bei diesem Test zeigen z.B. die folgenden Wirkstoffe eine gute Wirksamkeit: Nrn. 5, 6, 7, 10, 18, 19, 20, 23, 24, 52, 53, 54.

*Beispiel C:*

*Kontaktwirkung auf Kornkäfer* (Sitophilus granaria)

Petrischalen von 10 cm Durchmesser werden mit acetonischer Wirkstofflösung ausgekleidet. Nach dem Verdunsten des Lösungsmittels belegt man die Schalen mit 100 Kornkäfern. Nach 4 h werden die Käfer in unbehandelte Gefässe überführt. Die Mortalitätsrate wird nach 24 h ermittelt. Dabei wird festgestellt, wie viele Käfer in der Lage sind, nach diesem Zeitpunkt innerhalb 60 min ein unbehandeltes Pappschälchen (Durchmesser 40 mm, Höhe 10 mm) zu verlassen.

Die Mortalitätsrate der Kornkäfer, die mit den Wirkstoffen Nrn. 5, 7, 9, 10, 18, 23 und 57 behandelt wurden, ist hoch.

*Beispiel D:*

*Kontaktwirkung auf Stubenfliegen* (Musca domestica)*; Dauerkontakt*

Beide Teile einer Petrischale von 10 cm Durchmesser werden mit insgesamt 2 ml der acetonischen Wirkstofflösung ausgekleidet. Nach dem Verdunsten des Lösungsmittels (ca. 30 min) bringt man je 10 Fliegen in die Schalen. Die Mortalitätsrate wird nach 4 h festgestellt.

In diesem Test zeigen die Wirkstoffe Nrn. 4, 5, 6, 7, 9, 10, 20, 23, 24, 51, 52, 53 und 54 eine gute Wirkung.

*Beispiel E:*

*Zuchtversuch mit Stubenfliegen* (Musca domestica)

50 g eines Nährbodens aus
100 Teilen Wasser
10 Teilen Bäckerhefe
10 Teilen Trockenmilch, und
1 Teil Agar
werden in warmem Zustand mit der wässerigen Aufbereitung des Wirkstoffes gründlich durchmischt.

Nach dem Erkalten belegt man den Nährboden mit ca. 0,1 ml Fliegeneiern und beobachtet deren entwicklung über eine Woche. Die Versuchstemperatur liegt bei 20°C.

In diesem Test wird mit wässerigen Aufbereitungen der Wirkstoffe Nrn. 7, 9, 10 und 18 eine totale Hemmung der Entwicklung erzielt.

*Beispiel F:*

*Kontaktwirkung auf Baumwollwanzen* (Dysdercus intermedius)

Petrischalen von 10 cm Durchmesser werden mit 1 ml acetonischer Wirkstofflösung ausgekleidet. Nach dem Verdunsten des Lösungsmittels besetzt man die Schalen mit je 20 Larven des vorletzten Stadiums und registriert die Wirkung nach 24 h.

In diesem Test zeigen die Wirkstoffe Nrn. 4, 5, 6, 7, 24 und 26 eine gute Wirkung.

*Beispiel G:*

*Kontaktwirkung auf Blattläuse* (Aphis fabae)*; Spritzversuch*

Getopfte Bohnenpflanzen *(Vicia faba)* mit starken Blattlauskolonien werden in einer Spritzkammer mit wässerigen Wirkstoffaufbereitungen tropfnass gespritzt. Die Auswertung erfolgt nach 48 h.

In diesem Test zeigen die Verbindungen Nrn. 7 und 10 eine gute Wirkung.

*Beispiel H:*

*Frass- und Kontaktwirkung auf Raupen der Kohlschabe* (Plutella maculipennis)

Blätter von jungen Kohlpflanzen werden 3 s lang in die wässerige Wirkstoffemulsion getaucht und nach kurzem Abtropfen auf einen angefeuchteten Filter in eine Petrischale gelegt. Das blatt wird darauf mit 10 Raupen des 4. Stadiums belegt. Nach 48 h beurteilt man die Wirkung.

Mit den Verbindungen Nrn. 5, 6, 7, 9, 10, 18 und 26 wird in diesem Versuch eine sehr hohe Mortalität der Schädlinge erzielt.

*Beispiel J:*

*Kontaktwirkung auf Zecken* (Ornithodorus moubata)

Geprüft wird mit Zecken im 3. Larvenstadium. Dazu taucht man die Tiere, die sich in einem Papierbeutel befinden, für 3 s in die Prüfemulsion. Die Beutel werden frei aufgehängt. Nach 48 h wird die Wirkung auf die Zecken beurteilt.

In diesem Test wird mit Prüfemulsionen an Wirkstoffen der Formel I eine hohe Mortalitätsrate erzielt.

*Beispiel K:*

*Wirkung auf Spinnmilben* (Tetranychus telarius)

Getopfte Buschbohnen, die das erste Folgeblattpaar entwickelt haben und einen starken Besatz aller Stadien der Spinnmilbe *Tetranychus telarius* tragen, werden in der Spritzkabine mit der wässerigen Wirkstoffaufbereitung tropfnass gespritzt. Die Pflanzen kommen dazu auf einen Drehteller und werden von allen Seiten mit 50 ml Spritzbrühe besprüht. Der Sprühvorgang dauert ca. 22 s. Nach 8 d werden die Pflanzen auf lebende Spinmilben untersucht.

In diesem Test zeigen die Verbindungen Nrn. 5, 7 und 18 eine gute Wirksamkeit.

## Patentansprüche

1. Pyrazol-5-yl(thio)(thiol)phosphor(phosphon)säureester der Formel (I):

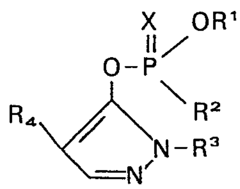

(I)

in der

X für Sauerstoff oder Schwefel,

$R^1$ für Alkyl mit 1 bis 4 Kohlenstoffatomen,

$R^2$ für Methyl, Äthyl. Phenyl oder für gegebenenfalls durch Halogen oder Alkoxy mit 1 bis 3 Kohlenstoffatomen substituiertes Alkoxy oder Alkylthio mit 1 bis 5 Kohlenstoffatomen,

$R^3$ für Wasserstoff, Alkyl mit 1 bis 6 Kohlenstoffatomen, Cycloalkyl mit 3 bis 6 Kohlenstoffatomen oder Phenyl, das durch Chlor, Brom, Methyl oder Alkoxy mit 1 bis 3 Kohlenstoffatomen substituiert sein kann, und

$R^4$ für Alkyl mit 1 bis 4 Kohlenstoffatomen stehen.

2. Verfahren zur Herstellung von Pyrazol-5-yl(thio)(thiol)phosphor(phosphon)säureestern der Formel (I) gemäss Anspruch 1, dadurch gekennzeichnet, dass man 5-Hydroxypyrazole der Formel (II):

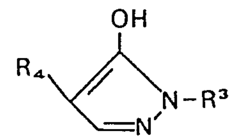

(II)

in der die $R^3$ und $R^4$ die im Anspruch 1 angegebenen Bedeutungen haben, in Gegenwart eines Säureakzeptors oder Salze dieser 5-Hydroxypyrazole mit (Thio)(thiol)phosphor(phosphon)esterhalogeniden der Formel (III):

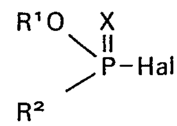

(III)

in der Hal Halogen bedeutet und $R^1$, $R^2$ und X die im Anspruch 1 angegebenen Bedeutungen haben, gegebenenfalls in Gegenwart eines organischen Lösungsmittels umsetzt.

3. Schädlingsbekämpfungsmittel, enthaltend einen Pyrazol-5-yl(thio)(thiol)phosphor(phosphon)säureester der Formel (I) gemäss Anspruch 1.

4. Schädlingsbekämpfungsmittel, enthaltend inerte Zusatzstoffe und einen Pyrazol-5-yl(thio)(thiol)phosphor(phosphon)säureester der Formel (I) gemäss Anspruch 1.

5. Insektizides und akarizides Mittel, enthaltend einen Pyrazol-5-yl(thio)(thiol)phosphor(phosphon)säureester der Formel (I) gemäss Anspruch 1.

6. Verfahren zur Bekämpfung von Schädlingen, dadurch gekennzeichnet, dass man eine wirksame Menge eines Pyrazol-5-yl(thio)(thiol)phosphor(phosphon)säureester der Formel (I) gemäss Anspruch 1 auf Schädlinge und/oder ihren Lebensraum einwirken lässt.

## Claims

1. A pyrazol-5-yl(thio)(thiol)phosphoric(phosphonic) acid ester of the formula (I):

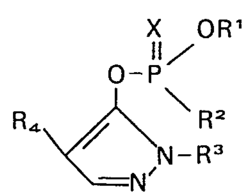

(I)

where:

X is oxygen or sulphur,

$R^1$ is alkyl of 1 to 4 carbon atoms,

$R^2$ is methyl, ethyl, phenyl, or alkoxy or alkylthio, each of 1 to 5 carbon atoms, which are unsubstituted or substituted by halogen or by alkoxy of 1 to 3 carbon atoms,

$R^3$ is hydrogen, alkyl of 1 to 6 carbon atoms, cycloalkyl of 3 to 6 carbon atoms or phenyl, which is unsubstituted or substituted by chlorine, bromine, methyl or alkoxy of 1 to 3 carbon atoms, and

$R^4$ is alkyl of 1 to 4 carbon atoms.

2. A process for the preparation of a pyrazol-5-yl(thio)(thiol)phosphoric(phosphonic) acid ester

of the formula (I) as claimed in claim 1, wherein a 5-hydroxypyrazole of the formula (II):

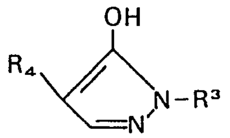

(II)

where $R^3$ and $R^4$ have the meanings given in claim 1, is reacted in the presence of an acid acceptor, or a salt of this 5-hydroxypyrazole is reacted, with a (thio)(thiol)phosphoric(phosphonic) acid ester halide of the formula (III):

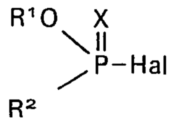

(III)

where Hal is halogen, and $R^1$, $R^2$ and X have the meanings given in claim 1, the reaction being carried out in the presence or absence of an organic solvent.

3. A pesticide containing a pyrazol-5-yl(thio)-(thiol)phosphoric(phosphonic) acid ester of the formula (I) as claimed in claim 1.

4. A pesticide containing inert additives and a pyrazol-5-yl(thio)(thiol)phosphoric(phosphonic) acid ester of the formula (I) as claimed in claim 1.

5. An insecticidal and acaricidal agent containing a pyrazol-5-yl(thio)(thiol)phosphoric(phosphonic) acid ester of the formula (I) as claimed in claim 1.

6. A process for controlling pests, wherein an effective amount of a pyrazol-5-yl(thio)(thiol)-phosphoric(phosphonic) acid ester of the formula (I) as claimed in claim 1 is allowed to act on pests and/or their habitat.

## Revendications

1. Esters d'acides pyrazolyl-5(thio)(thiol)-phosphoriques(phosphoniques) de formule (I):

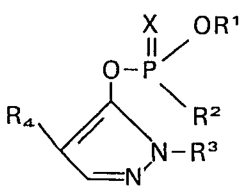

(I)

dans laquelle:

X désigne un atome d'oxygène ou de soufre,

$R^1$ un radical alcoyle en $C_1$ à $C_4$,

$R^2$ un groupe méthyle, éthyle ou phényle ou un groupe alcoxy ou alcoylthio en $C_1$ à $C_5$ éventuellement halo- ou alcoxy- (en $C_1$ à $C_3$) substitué.

$R_3$ un atome d'hydrogène, un groupe alcoyle en $C_1$ à $C_6$ ou cycloalcoyle en $C_3$ à $C_6$ ou un groupe phényle éventuellement chloro-, bromo-, méthyl- ou alcoxy- (en $C_1$ à $C_3$) substitué, et

$R^4$ un radical alcoyle en $C_1$ à $C_4$.

2. Procédé de préparation d'esters d'acides pyrazolyl-5(thio)(thiol)phosphoriques(phosphoniques) de la formule (I) selon la revendication 1, caractérisé en ce que l'on fait réagir un hydroxy-5-pyrazole de formule (II):

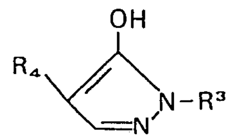

(II)

dans laquelle $R^3$ et $R^4$ possèdent les significations définies dans la revendication 1, en présence d'un fixateur d'acide, ou un sel d'un tel hydroxy-5-pyrazole, éventuellement dans un solvant organique, avec un halogénure d'ester d'acide (thio)-(thiol)phosphorique(phosphonique) de formule (III):

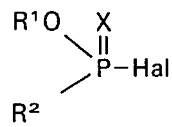

(II)

dans laquelle Hal est un halogène et $R^1$, $R^2$ et X possèdent les significations définies dans la revendication 1.

3. Composition pesticide, contenant un ester d'acide pyrazolyl-5(thio)(thiol)phosphorique-(phosphonique) de formule (I), selon la revendication 1.

4. Composition pesticide, contenant un ester d'acide pyrazolyl-5(thio)(thiol)phosphorique-(phosphonique) de formule (I), selon la revendication 1, ainsi que des additifs inertes.

5. Produit insecticide et acaricide, contenant un ester d'acide pyrazolyl-5(thio)(thiol)phosphorique(phosphonique) de formule (I), selon la revendication 1.

6. Procédé de lutte contre les parasites, caractérisé en ce que l'on fait agir une quantité efficace d'un ester d'acide pyrazolyl-5(thio)(thiol)phosphorique(phosphonique) de formule (I), selon la revendication 1, sur les parasites et/ou leur biotope.